(19) Europäisches Patentamt
European Patent Office
Office européen des brevets

(11) Veröffentlichungsnummer: **0 010 162**
**B1**

(12) **EUROPÄISCHE PATENTSCHRIFT**

(45) Veröffentlichungstag der Patentschrift: **25.11.81**

(51) Int. Cl.³: **C 07 C 143/70**

(21) Anmeldenummer: **79103457.2**

(22) Anmeldetag: **15.09.79**

(54) **Verfahren zur Herstellung von Alkansulfonsäurechloriden.**

(30) Priorität: **21.10.78 DE 2845918**

(43) Veröffentlichungstag der Anmeldung: **30.04.80 Patentblatt 80/9**

(45) Bekanntmachung des Hinweises auf die Patenterteilung: **25.11.81 Patentblatt 81/47**

(84) Benannte Vertragsstaaten: **DE FR GB**

(56) Entgegenhaltungen: **DE-A-1 811 768**

(73) Patentinhaber: **Merck Patent Gesellschaft mit beschränkter Haftung, Frankfurter Strasse 250, D-6100 Darmstadt (DE)**

(72) Erfinder: **Hübenett, Fritz, Dr., Herrngartenstrasse 37, D-6100 Darmstadt (DE)**

**EP 0 010 162 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Alkansulfonsäurechloriden

Die Erfindung betrifft ein Verfahren zur Herstellung von Alkansulfonsäurechloriden durch Umsetzung von Alkanthiolen und/oder Dialkyldisulfiden mit Chlor und Wasser.

Alkansulfonsäurechloride mit 1—12 Kohlenstoffatomen werden in großen Mengen als Ausgangsmaterialien und Zwischenprodukte bei organischen Synthesen, z. B. von Pflanzenschutzmitteln oder fotografischen Farbentwicklern gebraucht. Verschiedene Verfahren zu ihrer Herstellung, auch in technischem Maßstab, sind bekannt, wobei die Synthese aus Alkanthiolen bzw. Dialkyldisulfiden durch Umsetzung mit Chlor und Wasser bevorzugt ist. Die Nachteile dieses bekannten diskontiuierlichen Verfahrens liegen in noch unbefriedigenden Ausbeuten, in einer für viele Verwendungszwecke nicht ausreichenden Reinheit des Reaktionsproduktes und in der Notwendigkeit von mechanischen Rühreinrichtungen.

Darüber hinaus ist ein Verfahren zur kontinuierlichen Herstellung von Alkansulfonsäurechloriden, bei dem Alkanthiole bzw. Dialkyldisulfide in konzentrierter wäßriger Salzsäure mit Chlor in einem Umlaufreaktor umgesetzt werden, aus der DE-OS 1 811 768 bekannt. Die notwendige intensive Durchmischung der Reaktionspartner erfolgt dabei durch den bei der Reaktion gebildeten Chlorwasserstoff. Das spezifisch schwerere Alkansulfonsäurechlorid scheidet sich aus der als Reaktionsmedium dienenden wäßrigen Salzsäure ab, sobald diese bei Reaktionstemperatur damit gesättigt ist. Um eine ausreichend schnelle Abscheidung zu ermöglichen, muß intermittierend oder kontinuierlich ein Teil der mit dem Alkansulfonsäurechlorid gesättigten Salzsäure abgezogen und durch Wasser oder frische Salzsäure ersetzt werden; hierin liegt ein wesentlicher Nachteil dieses bekannten Verfahrens, weil das mit der Salzsäure abgezogene gelöste Alkansulfonsäurechlorid, das z. B. bei der Herstellung von Methansulfonsäurechlorid etwa 10 Gewichtsprozent des abgezogenen Reaktionsmediums ausmacht, nur durch eine aufwendige Aufarbeitung daraus abgetrennt werden kann. Ein Verzicht auf die Abtrennung verringert die Ausbeute und damit ebenfalls die Wirtschaftlichkeit des Verfahrens und erfordert zudem erhöhte Aufwendungen zur Unschädlichmachung im Interesse des Umweltschutzes.

Es stellte sich daher die Aufgabe, ein Verfahren zur Herstellung von Alkansulfonsäurechloriden zu entwickeln, bei dem dieses Produkt ohne Schwierigkeiten vollständig aus dem Reaktionsgemisch gewonnen werden kann.

Erfindungsgemäß wird diese Aufgabe dadurch gelöst, daß die Umsetzung der Alkanthiole oder Dialkyldisulfide mit Chlor und Wasser in dem als Reaktionsprodukt gewünschten Alkansulfonsäurechlorid vorgenommen wird.

Gegenstand der Erfindung ist somit ein Verfahren zur Herstellung von Alkansulfonsäurechloriden mit 1—12 Kohlenstoffatomen durch Umsetzung entsprechender Alkanthiole und/oder Dialkyldisulfide mit Chlor und Wasser bei einer Temperatur zwischen −10 und +50 C, das dadurch gekennzeichnet ist, daß das gewünschte Alkansulfonsäurechlorid als Reaktionsmedium dient.

Die bei dem erfindungsgemäßen Verfahren ablaufende Reaktion kann durch folgende Gleichungen wiedergegeben werden:

$$RSH + 3\,Cl_2 + 2\,H_2O \rightarrow R\,SO_2Cl + 5\,HCl$$

oder

$$(RS)_2 + 5\,Cl_2 + 4\,H_2O \rightarrow 2\,RSO_2Cl + 8\,HCl.$$

R bedeutet dabei eine Alkylgruppe mit 1—12 Kohlenstoffatomen.

Das erfindungsgemäße Verfahren kann chargenweise in jeder normalen Reaktionsapparatur durchgeführt werden. Zweckmäßiger erscheint es jedoch, eine kontinuierlich betriebene Umlaufapparatur zu verwenden. Üblicherweise arbeitet man bei Normaldruck. Die Reaktionstemperaturen liegen zwischen −10 und 50° C, vorzugsweise zwischen 25 und 35° C. Sowohl bei chargenweisem wie bei kontinuierlichem Arbeiten kann der bevorzugte Temperaturbereich unschwer durch einfache Wasserkühlung eingestellt werden; das Verfahren ist so mit geringstmöglichem Aufwand steuerbar. Höhere Reaktionstemperaturen z. B. von 40—50° C, sind dagegen zweckmäßig bei der Herstellung der Alkansulfonsäurechloride, die bei Raumtemperatur bereits in festem Zustand vorliegen.

Als Ausgangsmaterialien finden sowohl Alkanthiole als auch Dialkyldisulfide, bei denen die beiden Alkylgruppen einander gleich sind, Verwendung. In der Regel bevorzugt man bei der Herstellung der niederen Alkansulfonsäurechloride Dialkyldisulfide als Ausgangsmaterialien und zur Herstellung der höheren Derivate Alkanthiole als Ausgangsmaterialien. Selbstverständlich können die niederen Alkansulfonsäurechloride aber auch aus den Alkanthiolen hergestellt werden.

Chlor kann gasförmig oder in flüssiger Form eingeleitet werden. Wenn flüssges Chlor zur Verfügung steht, ist die Einspeisung in dieser Form insbesondere bei größeren Anlagen zweckmäßig, weil ein Teil der Reaktionswärme als Verdampfungswärme abgeführt wird, so daß weniger Kühlung notwendig ist.

Die Reaktanten werden in der Regel getrennt in das Reaktionssystem eingespeist. Prinzipiell ist es aber auch möglich, das Alkanthiol bzw. das Dialkyldisulfid in Form einer Emulsion mit Wasser einzusetzen, wobei zur Herstellung einer solchen Emulsion auch oberflächen-aktive Substanzen in sehr geringen Konzentrationen als Emulgatoren zugesetzt werden können.

In der Regel werden die Reaktanten in stöchiometrischen oder wenigstens annähernd stöchiometrischen Mengen eingesetzt. Es hat sich als zweckmäßig erwiesen, einen geringen Chlorüberschuß zu verwenden. Dadurch werden Spuren von Schwefelverbindungen, die durch die starke Gasentwicklung mitgerissen werden können, in der nachgeschalteten adiabatisch betriebenen Absorptionskolonne oxidiert. In der als Nebenprodukt anfallenden Salzsäure sind so nach Neutralisation übelriechende Schwefelverbindungen mit niedrigen Oxidationsgraden nicht nachweisbar.

Das erfindungsgemäße Verfahren bietet besondere Vorteile, wenn es kontinuierlich in einem üblichen Umlaufreaktor durchgeführt wird, wobei die Durchmischung der Reaktionsteilnehmer durch den bei der Reaktion entstehenden Chlorwasserstoff erfolgt. Eine bevorzugte Ausführungsform einer Anlage, in der das erfindungsgemäße Verfahren durchgeführt werden kann, ist in Fig. 1 dargestellt. Die Anlage besteht aus einem Umlaufreaktor (1) und einer Reinigungsvorrichtung (2) für das hergestellte Alkansulfonsäurechlorid; es hat sich als zweckmäßig erwiesen, zusätzlich noch ein Absorptionssystem (3) für den bei der Reaktion entstehenden Chlorwasserstoff vorzusehen. Der Umlaufreaktor (1) besteht aus der Reaktionszone (11), dem Kühler (12), dem Gasabscheider (13) und der Umlaufleitung (14). In die Reaktionszone (11) münden die Zuleitungen: (15) für Chlor, (16) für Alkanthiol bzw. Dialkyldisulfid und (17) für Wasser. Die eingezeichnete Anordnung der Zuleitungen hat sich für die technische Durchführung des erfindungsgemäßen Verfahrens als besonders vorteilhaft erwiesen, weil dadurch Verluste an nicht umgesetzten Alkanthiol bzw. Dialkyldisulfid besonders gering gehalten werden; prinzipiell können jedoch auch beispielsweise die Zuleitung (15) für Alkanthiol bzw. Dialkyldisulfid und die Zuleitung (16) für Chlor verwendet werden, oder alle drei Zuleitungen (15), (16) und (17) einander benachbart in der Reaktionszone (11) münden. Der Kühler (12) kann prizipiell von beliebiger Bauart sein; er muß lediglich so dimensioniert sein, daß die Reaktionswärme abgeführt werden kann; die Verwendung eines Röhrenkühlers hat sich als besonders zweckmäßig erwiesen. Als Gasabscheider (13), in dem die Reaktionsprodukte Alkansulfonsäurechlorid und Chlorwasserstoff getrennt werden, kommen ebenfalls alle herkömmlichen Typen in Frage; vorzugsweise werden Zyklone oder Aufprallabscheider verwendet. Das abgetrennte Alkansulfonsäurechlorid fließt teilweise durch die Umlaufleitung (14) wieder in die Reaktionszone (11), teils durch die Ableitung (21) in die Reinigungsvorrichtung (2). Diese Reinigungsvorrichtung (2) kann beispielsweise eine Blasensäule sein, in der dem Alkansulfonsäurechlorid durch die Zuleitung (22) im Gegenstrom Luft oder ein Inertgas wie Stickstoff entgegengeführt wird, um in dem Produkt gelösten Chlorwasserstoff auszutreiben. Durch die Ableitung (24) wird das so gereinigte Alkansulfonsäurechlorid dem Behälter für das Endprodukt zugeführt. Das Abgas der Blasensäule wird durch die Leitung (23)/(33) zusammen mit dem aus dem Gasabscheider durch die Ableitung (19)/(33) entweichenden Chlorwasserstoff dem Absorptionssystem (3) zugeführt. Anstelle einer Blasensäule können als Reinigungsvorrichtung (2) auch beispielsweise eine mit vermindertem Druck betriebene Rieselkolonne oder ein Kessel zur Vakuumentgasung angewendet werden. Das Absorptionssystem (3) ist in allgemein üblicher Weise ausgeführt, wobei die Leistungsfähigkeit wesentlich von im Interesse des Umweltschutzes zu stellenden Anforderungen abhängt.

Zur kontinuierlichen Durchführung des erfindungsgemäßen Verfahrens wird der Umlaufreaktor (1) mit einer solchen Menge des herzustellenden Alkansulfonsäurechlorids beschickt, daß nach dem folgenden Einführen von Chlor, Alkanthiol bzw. Dialkyldisulfid und Wasser und damit dem Beginn der Bildung des Alkansulfonsäurechlorids der Umlauf sofort in Gang kommt. Dabei werden die entsprechenden Zuleitungen zweckmäßig in der vorgenannten Reihenfolge geöffnet, um möglichst gleich zu Anfang ein von flüchtigen und übelriechenden Schwefelverbindungen freies Abgas zu erhalten.

Nach dem erfindungsgemäßen Verfahren werden Alkansulfonsäurechloride mit 1—12 C-Atomen in höchster Reinheit, d. h. mit deutlich weniger als 1% an Verunreinigungen, und mit ausgezeichneten Ausbeuten erhalten. Bevorzugt wird das Verfahren zur Herstellung von bei Raumtemperatur flüssigen Alkansulfonsäurechloriden verwendet, insbesondere zur Herstellung von Methansulfonsäurechlorid.

Überraschenderweise bestehen die bei dem erfindungsgemäßen Verfahren in Gasform anfallenden Produkte neben Luft bzw. Inertgas, die im Reinigungsvorgang eingeblasen werden, aus so reinem Chlorwasserstoff, daß sie in der Absorptionsvorrichtung zur Herstellung einer sehr reinen wäßrigen Salzsäure verwenden werden können. Diese Salzsäure weist nur Verunreinigungen von weniger als 0,2% auf. Wird das erfindungsgemäße Verfahren zusätzlich mit einem geringen Chlorüberschuß durchgeführt, so sind in der als Nebenprodukt anfallenden Salzsäure nach Neutralisation Schwefelverbindungen auch durch den Geruch nicht nachweisbar.

Die folgenden Beispiele sollen die Erfindung erläutern, ohne sie zu begrenzen.

### Beispiel 1

In einer der Fig. 1 entsprechenden Apparatur werden 3504 g Methansulfonsäurechlorid eingefüllt. Im Lauf von 24 Stunden werden 3956 g Dimethyldisulfid in Form einer mit 21 ml eines handelsüblichen Nonylphenolpolyglykoläthers hergestellten Emulsion mit 3026 g Wasser eingespeist und 15,19 kg Chlorgas mit einer Geschwindigkeit von 220—240 Liter pro Stunde eingeblasen. In der als Reinigungsvorrichtung (2) dienenden, mit Raschig-Ringen gefüllten Blasensäule wird Luft zum Ausblasen des anfallenden Methansulfonsäurechlorids durch die Leitung (22) eingeführt. Die Kühlung erfolgt durch einen Intensivkühler; die Reaktionstemperatur beträgt 29 C. In 24 Stunden werden — abzüglich der Menge des als Reaktionsmedium vorgelegten Methansulfonsäurechlorids — 9531 g Methansulfonsäurechlorid (99% d. Th.) mit einem gaschromatographisch bestimmten Reinheitsgrad von 99,6% abgezogen. Aus dem Absorptionssystem (3) werden in dieser Zeit 39,2 kg einer wäßrigen 30,3%igen Salzsäure (97% d. Th.) in höchster Reinheit erhalten. Die Salzsäure enthält als Verunreinigungen nur 0,2% Methansulfonsäure, < 1 ppm freies Chlor und < 5 ppm Sulfationen.

Beispiel 2

In eine Apparatur analog Beispiel 1 werden 2460 g auf 50°C erwärmtes Dodecansulfonsäurechlorid eingefüllt, wobei zur Verhinderung der Kristallisation auch die Umlaufleitung (14) und die Blasensäule (2) durch Heizmanschetten auf dieser Temperatur gehalten werden. Im Lauf von 5 Stunden werden 1518 g Dodecylmerkaptan in Form einer Emulsion mit 270 g Wasser (Emulgator: 2 ml handelsübliche Nonylphenolpolyglykoläther) eingespeist. Gleichzeitig werden 115 Liter Chlorgas pro Stunde eingeleitet. Es werden 1976 g Dodecansulfonsäurechlorid (F 42 C; 98% d. Th.) und 4,2 kg einer wäßrigen 31,5%igen Salzsäure erhalten.

Beispiel 3

In einer aus technischem Glas erstellten Anlage mit einem Kühler (12), einer Blasensäule (2) von 3,0 m Länge und einer HCl-Absorptionskolonne (3) von 4,0 m Länge werden stündlich 13,65 Liter Dimethyldisulfid, 11,0 Liter Wasser und 55,4 kg flüssiges Chlor eingespeist. Die Reaktionstemperatur wird auf 30 C gehalten. Durch die Ableitung (24) werden 34,8 kg Methansulfonsäurechlorid pro Stunde (99,2% d. Th.) abgezogen, die nach gaschromatographischer Analyse einen Reinheitsgrad von 99,4% besitzen. Zugleich werden aus der Absorptionskolonne stündlich 38,1 kg einer wäßrigen 30,8%igen Salzsäure in höchster Reinheit erhalten. Die Salzsäure enthält als Verunreinigungen nur 0,1% Methansulfonsäure und 2 ppm freies Chlor.

## Patentansprüche

1. Verfahren zur Herstellung von Alkansulfonsäurechloriden mit 1—12 C-Atomen durch Umsetzung entsprechender Alkanthiole und/oder Dialkyldisulfide mit Chlor und Wasser bei einer Temperatur zwischen —10 und +50°C, dadurch gekennzeichnet, daß das gewünschte Alkansulfonsäurechlorid als Reaktionsmedium dient.

2. Verfahren nach Anspruch 1, dadurch gekennzeichnet, daß die Umsetzung kontinuierlich in einem Umlaufreaktor durchgeführt wird, wobei der Umlauf durch den bei der Reaktion gebildeten Chlorwasserstoff bewirkt wird.

3. Verfahren nach den Ansprüchen 1 und 2, dadurch gekennzeichnet, daß die Umsetzung bei 25—35 C mit einem geringen Überschuß an Chlor und nicht mehr als der stöchiometrischen Menge Wasser durchgeführt wird.

## Claims

1. Process for the preparation of alkanesulphonic acid chlorides having 1—12 C atoms by reaction of corresponding alkanethiols and/or dialkyl disulphides with chlorine and water at a temperature of between —10 and +50 C, characterised in that the desired alkanesulphonic acid chloride serves as the reaction medium.

2. Process according to Claim 1, characterised in that the reaction is carried out continuously in a circulatory reactor, the circulation being effected by the hydrogen chloride formed in the reaction.

3. Process according to Claims 1 and 2, characterised in that the reaction is carried out at 25—35 C with a slight excess of chlorine and not more than the stoichiometric amount of water.

## Revendications

1. Procédé de préparation de chlorures d'acides alcane-sulfoniques en $C_1$ à $C_{12}$ par réaction d'alcane-thiols et/ou de disulfure de dialcoyles correspondants avec du chlore et de l'eau à une température comprise entre —10 et +50° C, caractérisé en ce que le chlorure d'acide alcane-sulfonique désiré sert de milieu réactionnel.

2. Procédé selon la revendication 1, caractérisé en ce que la réaction s'effectue en continu dans un réacteur à recirculation, le recyclage étant provoqué par l'acide chlorhydrique formé dans la réaction.

3. Procédé selon les revendications 1 et 2, caractérisé en ce que la réaction est conduite à 25—35 C avec un léger excès de chlore et pas plus de la quantité stoechiométrique d'eau.

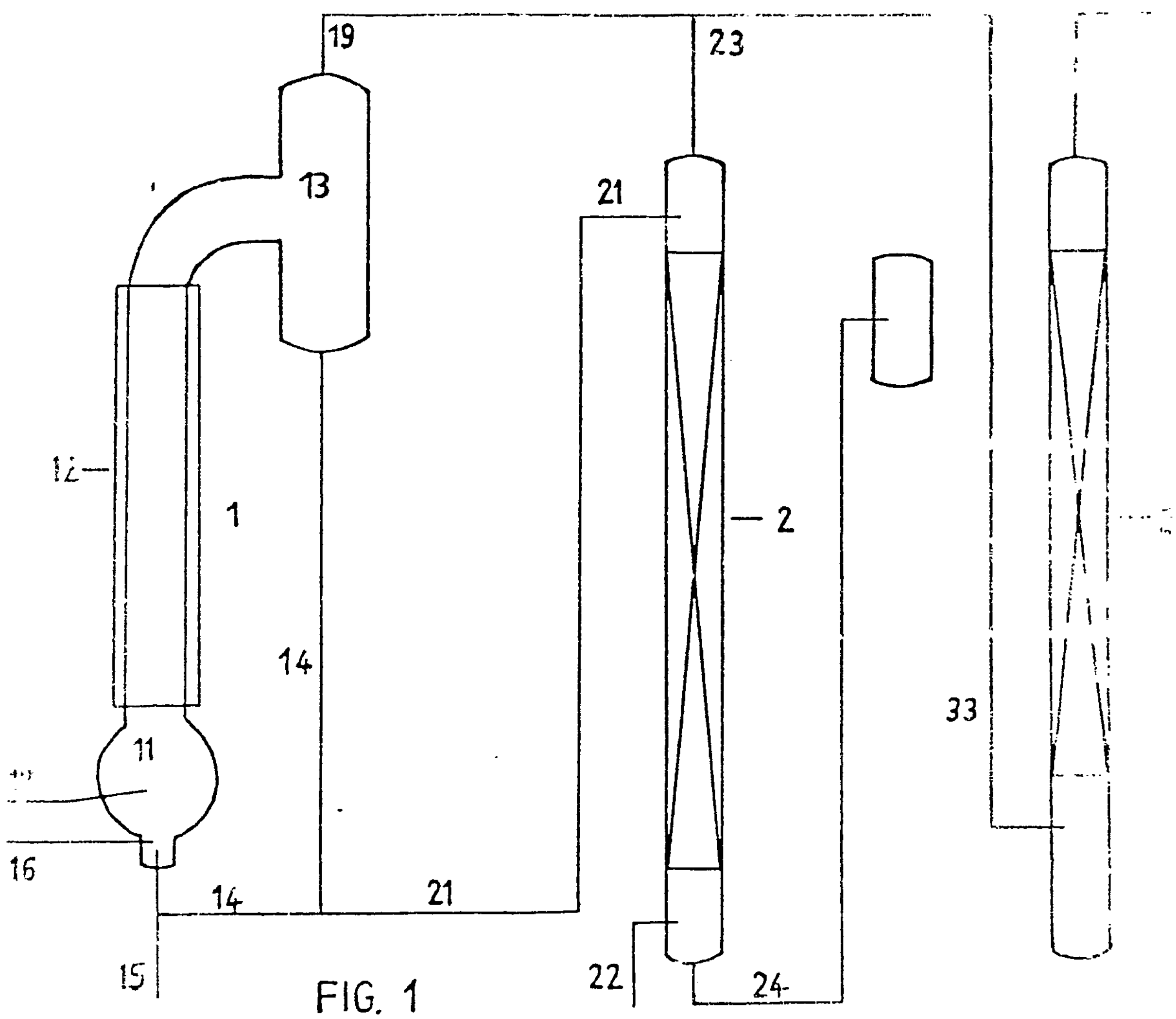


FIG. 1